# EUROPEAN PATENT APPLICATION

(11) **EP 4 640 232 A1**
(43) Date of publication of application: **29.10.2025**
(21) Application number: 23905064.4
(22) Date of filing: 22.03.2023
(51) Int. Cl.: A61K 38/16, A61K 45/06, A61K 31/4418, A61P 35/00

(54) **USE OF RECOMBINANT PROTEIN IN PREPARING DRUG FOR INHIBITING ANEURYSM**

(30) Priority: 22.12.2022 CN 202211657323
(71) Applicant: Huazhong University of Science and Technology, Wuhan, Hubei 430074 (CN)
(72) Inventor: WANG, Qing, Wuhan, Hubei 430074 (CN); YAO, Yufeng, Wuhan, Hubei 430074 (CN); DA, Xingwen, Wuhan, Hubei 430074 (CN)
(74) Representative: Straus, Alexander
(86) International application number: PCT/CN2023/083061
(87) International publication number: WO 2024/130872

(57) **Abstract**

A use of a recombinant protein in preparing a drug for inhibiting aneurysm, which belongs to the field of biomedicine. Provided is a recombinant protein AGGF1 for use in preparing a drug for inhibiting aneurysm. The recombinant AGGF1 protein, by means of a receptor integrin a7 thereof, can inhibit the TGF-β1 signaling pathway and vascular remodeling. Therefore, the recombinant AGGF1 protein inhibits aneurysm by inhibiting TGF-B1 synthesis and a signaling pathway thereof, and the recombinant protein achieves the inhibition of aneurysm by improving the structure and functions of blood vessels.

## Description

### [Technical Field]

The present disclosure belongs to the field of biomedicine, more specifically, relates to a use of a recombinant protein in preparing drugs for inhibiting an aneurysm.

### [Description of Related Art]

An aneurysm is a disease characterized by localized or diffuse dilation or protrusion of a blood vessel due to pathological changes or damages to a vessel wall. The primary characteristics of the aneurysm include phenotypic transformation of vascular smooth muscle cells, vascular dilation, and hemodynamic alterations. The aneurysm may occur at any site; however, the sites where the aneurysm commonly occurs and causes serious damages are cerebral arteries and major arteries such as the aorta. The mortality rate following rupture of the aneurysm in these sites is exceedingly high. The etiology of the aneurysm is multifactorial, including chronic conditions such as atherosclerosis and hypertension, which may lead to deterioration of the vessel wall. Nevertheless, therapeutic options for the aneurysm remain limited; surgical intervention may result in adverse outcomes such as rupture or secondary infection, and effective pharmacological treatments are currently unavailable.

### [SUMMARY]

In view of the above deficiencies or need of improvement in the current technology, the present disclosure provides a use of a recombinant protein, with the purpose of preparing anti-aneurysm drugs through AGGF1 recombinant protein, effectively controlling the aggravation of an aneurysm and clinical interventions.

According to the purpose of the present disclosure, a use of a recombinant protein is provided, applied for preparing drugs for inhibiting the aneurysm; an amino acid sequence of the recombinant protein is as follows:
(1) As shown by an amino acid sequence of SEQ ID NO: 1; or
(2) An amino acid sequence having 80% to 100% homology with the amino acid sequence defined by SEQ ID NO: 1, and retaining the amino acids at positions 606-608, wherein the amino acid sequence encodes a protein with the same function; or
(3) An amino acid sequence defined by SEQ ID NO: 1 that has undergone addition, deletion, or substitution of one or more amino acids, and that retains the amino acids at positions 606-608, possessing an equivalent activity to the protein shown in the sequence of SEQ ID NO: 1.

According to another aspect of the present disclosure, a use of a composition of a recombinant protein with a TGF-β inhibitor is provided, applied for preparing drugs for inhibiting the aneurysm; an amino acid sequence of the recombinant protein is as follows:
(1) As shown by an amino acid sequence of SEQ ID NO: 1; or
(2) An amino acid sequence having 80% to 100% homology with the amino acid sequence defined by SEQ ID NO: 1, and retaining the amino acids at positions 606-608, wherein the amino acid sequence encodes a protein with the same function; or
(3) An amino acid sequence defined by SEQ ID NO: 1 that has undergone addition, deletion, or substitution of one or more amino acids, and that retains the amino acids at positions 606-608, possessing the equivalent activity to the protein shown in the sequence of SEQ ID NO: 1;

Preferably, the TGF-β inhibitor is pirfenidone.

Preferably, the aneurysm is an aortic aneurysm or a carotid artery aneurysm.

Preferably, the aneurysm is an aneurysm caused by aortic arch coarctation.

Preferably, the aneurysm is an aneurysm caused by FBN1 gene mutation.

Preferably, the aneurysm is a thoracic aortic aneurysm.

Preferably, the recombinant protein inhibits the aneurysm through inhibiting TGF-β1 synthesis and a signaling pathway thereof.

Preferably, the signaling pathway is TGFβ1-TGFβR1-Smad2/3 and TGFβ1-TGFβR1-ERK1/2.

Preferably, the recombinant protein inhibits the aneurysm through improving the structure and functions of blood vessels.

Preferably, the deletion of amino acids at the positions 606-608 disrupts an interaction between an AGGF1 protein and a receptor integrin α7 thereof, thereby eliminating an effect of the AGGF1 protein.

Overall, compared with the current technology, the technical solutions conceived by the present disclosure mainly possess the following technical advantages.
(1) The present disclosure provides a convenient drug administration method. In mouse experiments, the protein may exert its effect through intraperitoneal injections.
(2) The present disclosure shows significant effects. In protein treatment experiments, the therapeutic effect of a protein injection group is very significant, with blood vessel lesions almost completely restored to normal levels, effectively improving the aneurysm caused by vascular lesions.
(3) The present disclosure avoids the occurrence of post-surgery complications. Protein treatment relies only on injection without requiring surgical repair, thus avoiding adverse reactions such as vascular inflammations, aneurysm ruptures, etc.
(4) The recombinant AGGF1 protein provided by the present disclosure may improve vascular smooth muscles, inhibit vascular inflammations, and treat an aortic aneurysm and a carotid aneurysm through inhibiting a TGF-β1 synthesis and a TGF-β1 signaling pathway (Smads and ERK1/2).
(5) In the present disclosure, AGGF1 is required for pirfenidone to exert its therapeutic effect, which not only reflects the importance of AGGF1, but also expands the application of AGGF1 and pirfenidone in treating the aneurysm. Therefore, the recombinant AGGF1 protein with His tag in the present disclosure may be used to prepare anti-aneurysm drugs for the treatment of aneurysm diseases.
(6) The present disclosure simultaneously verified therapeutic effects of AGGF1 on thoracic aortic aneurysm-Marfan syndrome mice caused by FBN1 gene mutation and β-aminopropionitrile-induced aneurysm model, with good application effects and a wide range of applications.

### [BRIEF DESCRIPTION OF THE DRAWINGS]

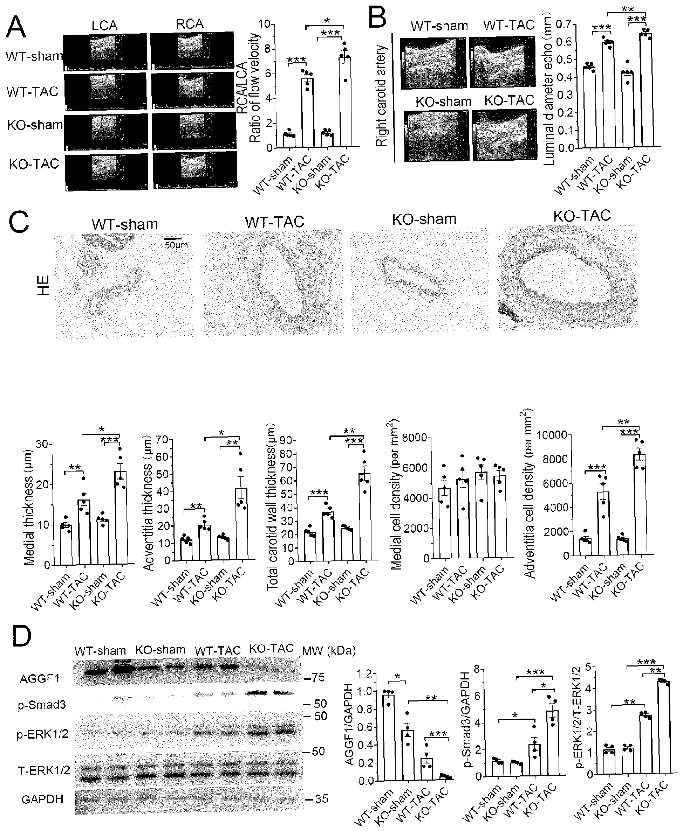

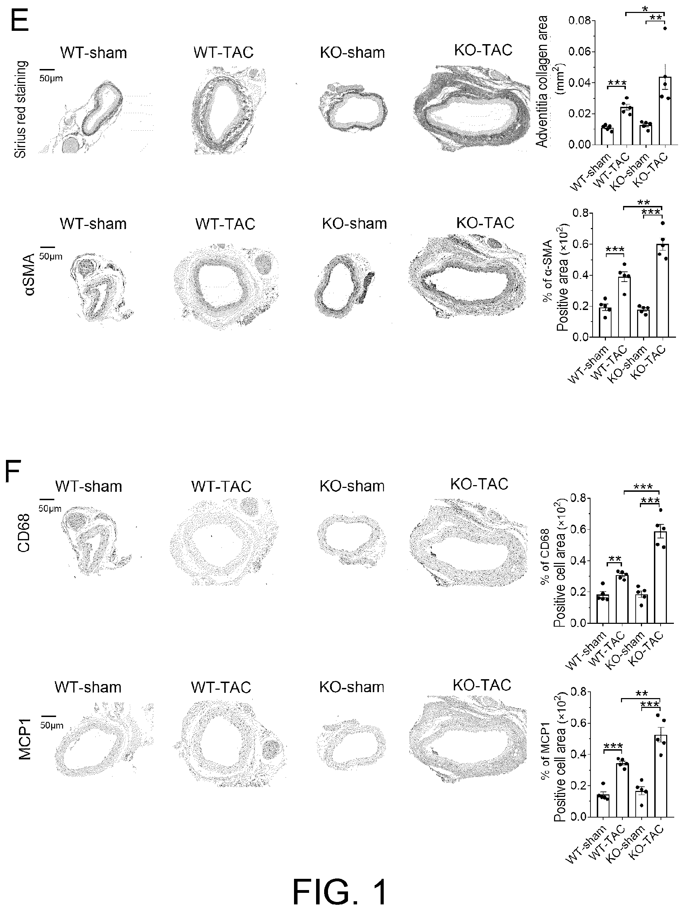

FIG. 1 shows that Aggfl haploinsufficiency (heterozygous Aggfl ^{+/-}) exacerbates TAC (Transverse Aortic Constriction)-induced right carotid artery dilation, remodeling, and inflammation. A model of aneurysm-associated arterial remodeling is established through a transverse aortic constriction (TAC) surgery. FIG. (A) shows echocardiographic images of blood flow velocities of a left carotid artery (LCA) and a right carotid artery (RCA) from Aggfl ^{+/+} (WT) or Aggfl^{+/-} (KO) mice three weeks after TAC or a sham surgery. A bar graph on the right shows a ratio of flow velocity (FV) between the RCA and the LCA. FIG. (B) shows echocardiographic images of the RCA from WT or KO mice three weeks after the surgery, with the bar graph on the right showing a luminal diameter of the RCA. RCAs are harvested after 21 days for a histological staining and a western blot analysis. FIG. (C) shows an H&E staining of cross-sections of the RCA. Bar graphs display a medial thickness (1st graph), an adventitia thickness (2nd graph), a total carotid wall thickness (3rd graph), a medial cell density (4th graph), and an adventitia cell density (5th graph). FIG. (D) shows a western blot analysis of AGGFl, phosphorylated ERK1/2 (p-ERK1/2), total ERK1/2 (T-ERK1/2), and phosphorylated Smad3 (p-Smad3). GAPDH serves as a loading control. Blots are quantified and data is plotted as bar graphs on the right. FIG. (E) shows a Sirius red staining and an immunostaining of a α-smooth muscle actin (α-SMA) in the cross-sections of the RCA. Bar graphs on the right show a quantification of a collagen area and a percentage of a α-SMA positive area. FIG. (F) shows an immunostaining of an inflammatory marker MCP-1 and a macrophage marker CD68 in the cross-sections of the RCA. Bar graphs on the right show a quantification of a percentage of a MCP1-positive cell area and a CD68-positive cell area. *, P<0.05; **, P<0.01; ***, P<0.001; a mouse sample size n=5/group, other sample sizes n=4/group.

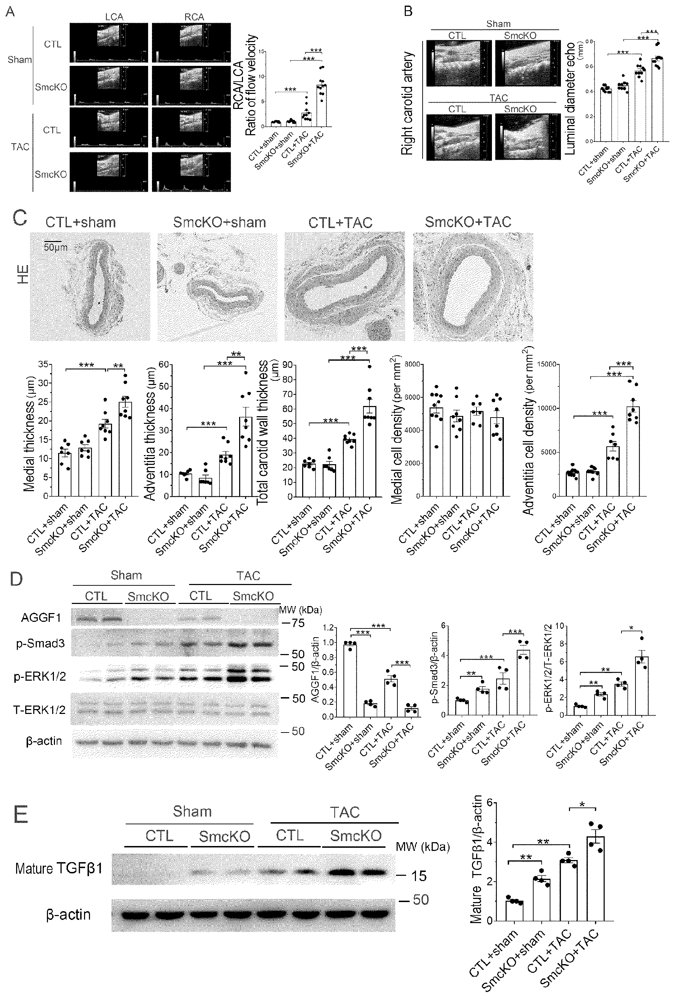

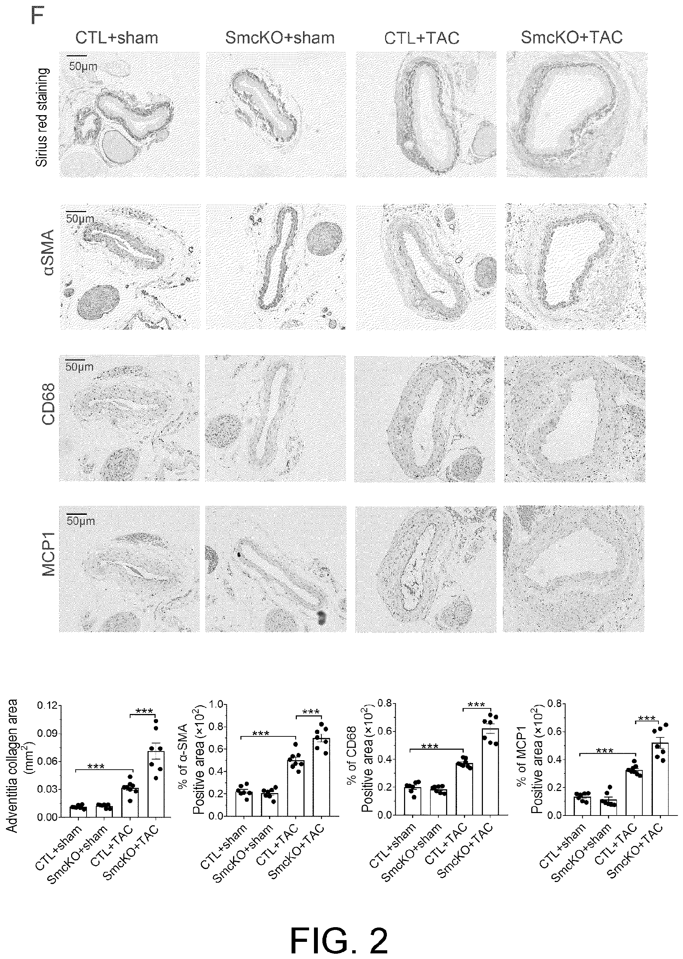

FIG. 2 demonstrates that a vascular smooth muscle-specific knockout of Aggfl (SmcKO) exacerbates the TAC-induced right carotid artery dilation, remodeling, and inflammation. FIG. (A) shows echocardiographic images of LCA and blood flow velocities of the RCA from Aggfl^{flox/flox} (CTL) and Aggfl^{smcKO} (SmcKO) mice three weeks after TAC or the sham surgery. The bar graph on the right illustrates a ratio of flow velocity (FV) between the RCA and the LCA. FIG. (B) shows echocardiographic images of the RCA from CTL or SmcKO mice three weeks after the surgery, with the bar graph on the right showing the luminal diameter of the RCA. RCAs are harvested after 21 days for a histological staining and a western blot analysis. FIG. (C) shows the H&E staining of the cross-sections of the RCA. Bar graphs below display the medial thickness (1st graph), the adventitia thickness (2nd graph), the total carotid wall thickness (3rd graph), the medial cell density (4th graph), and an adventitia cell density (5th graph) of a vessel. FIG. (D) shows a protein blot analysis of AGGF1, p-ERK1/2, T-ERK1/2, and p-Smad3. The β-actin serves as a loading control. FIG. (E) shows a protein blot analysis of a mature TGF-β1 in RCA. The β-actin serves as a loading control. FIG. (F) shows a Sirius red staining and an immunostaining of α-SMA, MCP-1 and CD68 in the RCA cross-sections. Bar graphs at the bottom show a percentage of the positive area for respective immunostaining quantifications. *, P<0.05; **, P<0.01; ***, P<0.001; a mouse sample size n=5/group, other sample sizes n=4/group.

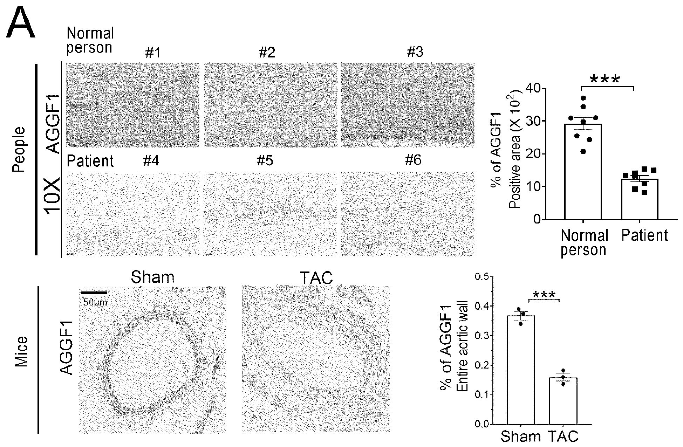

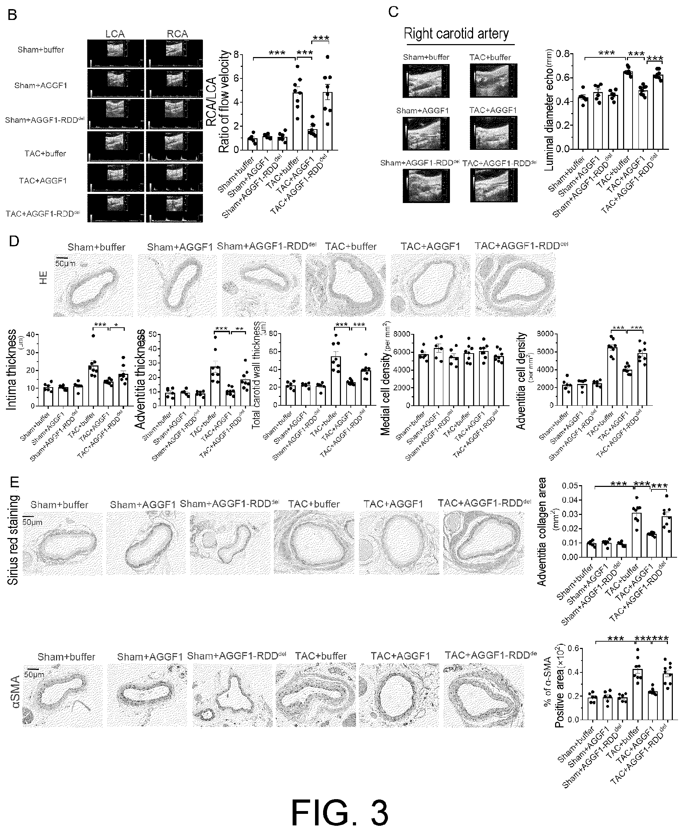

FIG. 3 shows that an AGGF1 protein expression level is significantly reduced in an arterial aneurysm mouse model and vascular tissues of a patient with the thoracic aortic aneurysm, while an intraperitoneal injection of a purified human AGGF1 protein, dependent on an RDD sequence, mitigates the TAC-induced right carotid artery dilation and remodeling in mice. In FIG. (A), an upper graph shows the AGGF1 immunostaining of vascular samples from 8 normal humans and 8 patients with the thoracic aortic aneurysm, and the lower graph shows the AGGF1 immunostaining of blood vessels from a Sham group and a TAC group. FIG. (B) shows echocardiographic images of a blood flow velocity of the LCA and the RCA in mice after TAC or the sham surgery with or without a wild-type human source AGGF1 or a mutant AGGF1-RDD^{del} treatment for three weeks. The bar graph on the right shows a ratio of the flow velocity (FV) between the RCA and the LCA. FIG. (C) shows the luminal diameter of the RCA. RCAs are harvested after 21 days for the histological staining. FIG. (D) shows the H&E staining of the cross-sections of the RCA. The bar graphs at the bottom show the intima thickness (1st graph), the adventitia thickness (2nd graph), the total arterial wall thickness (3rd graph), a media cell density (4th graph), and the adventitia cell density (5th graph). FIG. (E) shows the Sirius red staining and a α-SMA immunostaining of the cross-sections of the RCA. The bar graphs on the right show the percentage of the collagen area and the percentage of the α-SMA positive area. *, P<0.05; **, P<0.01; ***, P<0.001; a mouse sample size n≧6/group.

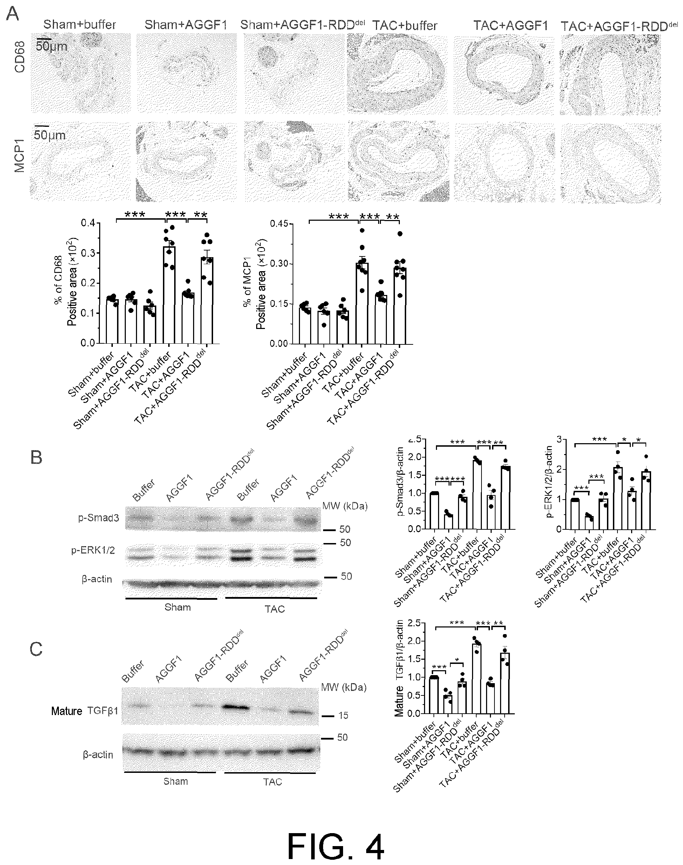

FIG. 4 demonstrates that the intraperitoneal injection of a purified human AGGF1 protein, dependent on the RDD sequence, attenuates a TAC-induced vascular inflammation, a TGF-β1 maturation and a signaling, and an ERK1/2 signaling in the right carotid artery of mice. RCAs are harvested after 21 days for the histological staining and the western blot analysis. FIG. (A) shows the immunostaining for MCP-1 and CD68 in the RCA cross-sections from the model in FIG. 3. Bar graphs below show the percentage of the MCP1-positive cell area and the CD68-positive cell area. FIG. (B) shows the western blot analysis of p-ERK1/2 and p-Smad3 in RCA. The β-actin serves as a loading control. FIG. (C) shows the western blot analysis of a mature TGF-B1 protein in RCA. The β-actin serves as a loading control. *, P<0.05; **, P<0.01; ***, P<0.001; a mouse sample size n≧6/group, other sample sizes n=4/group.

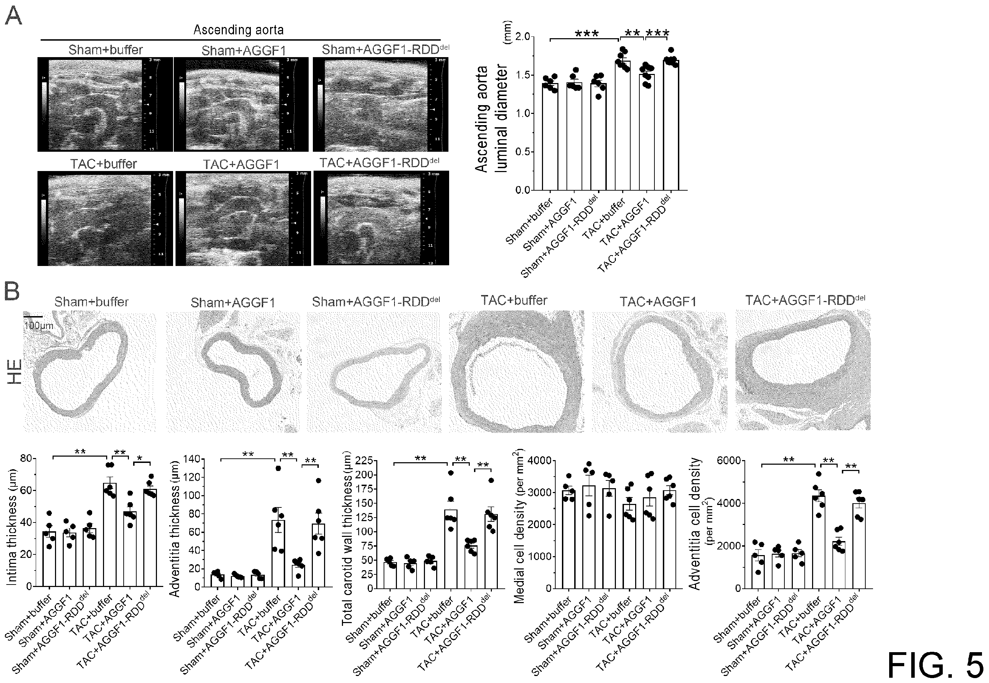

FIG. 5 demonstrates that the intraperitoneal injection of a purified human AGGF1 protein, dependent on an RDD sequence, alleviates a TAC-induced ascending aortic dilation and a remodeling in mice. FIG. (A) shows echocardiographic images of the ascending aorta from mice subjected to TAC or the sham surgery, with or without treatment with a wild-type human AGGF1 or a mutant AGGF 1-RDDdel for three weeks. The bar graph on the right illustrates the luminal diameter of the ascending aorta. An ascending aortic tissue is harvested after 21 days for the histological staining. FIG. (B) shows the H&E staining of the cross-sections of the RCA. The bar graphs below display the intimal thickness (1st graph), the adventitia thickness (2nd graph), the total arterial wall thickness (3rd graph), the medial cell density (4th graph), and the adventitia cell density (5th graph). *, P<0.05; **, P<0.01; ***, P<0.001; a mouse sample size n≧6/group.

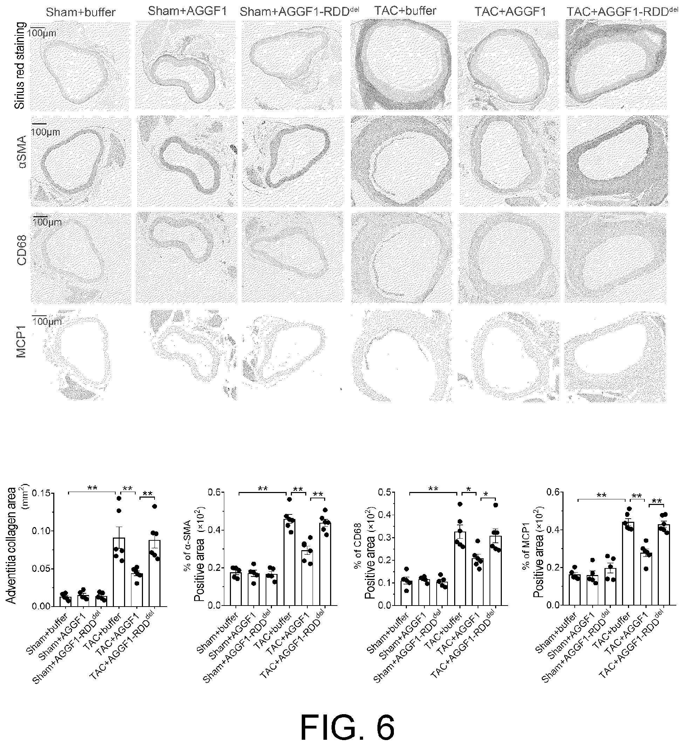

FIG. 6 demonstrates that the intraperitoneal injection of the purified human AGGF1 protein, dependent on the RDD sequence, may mitigate the TAC-induced ascending aortic remodeling and inflammation in mice. The ascending aortic tissue is harvested for the histological staining 21 days after the surgery. The figure sequentially displays, from top to bottom, the Sirius red staining of ascending aortic cross-sections, and the immunostaining for α-SMA, CD68, and MCP-1. Below are quantitative bar graphs showing the quantitation of the percentage of the positive area for each corresponding stain. *, P<0.05; **, P<0.01; ***, P<0.001; a mouse sample size n≧6/group.

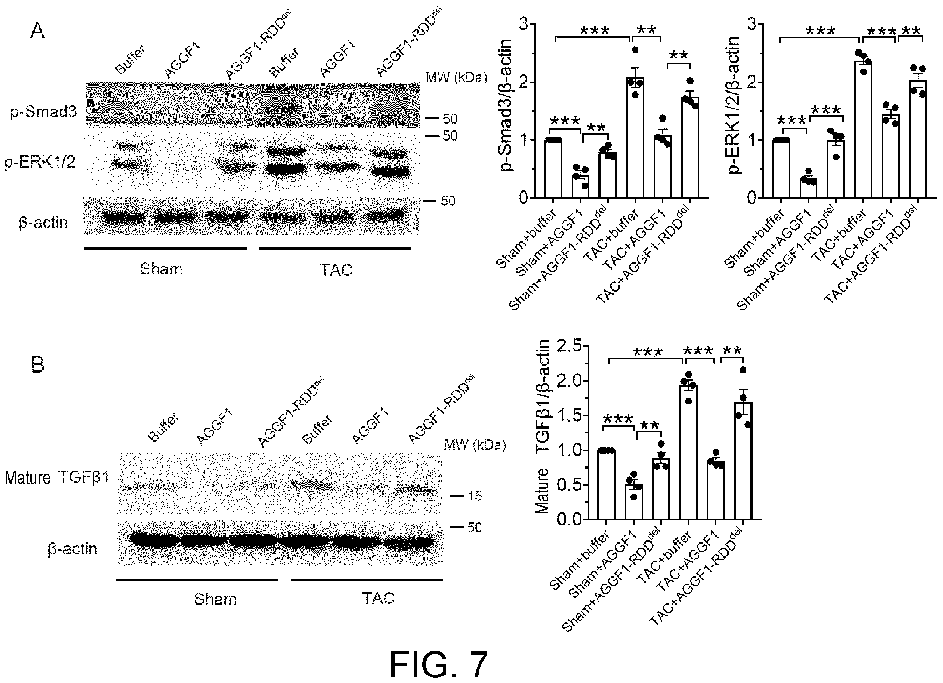

FIG. 7 depicts that the intraperitoneal injection of the purified human AGGF1 protein, dependent on the RDD sequence, attenuates a TAC-induced ascending aortic TGF-β1 maturation and a signaling pathway, as well as the ERK1/2 signaling pathway. The RCAs are harvested after 21 days for the western blot analysis. FIG. (A) shows the western blot analysis of p-ERK1/2 and p-Smad3 in an ascending aorta. The β-actin serves as a loading control. FIG. (B) shows the western blot analysis of the mature TGF-β1 protein in the ascending aorta. The β-actin serves as a loading control. *, P<0.05; **, P<0.01; ***, P<0.001; sample size n=4/group.

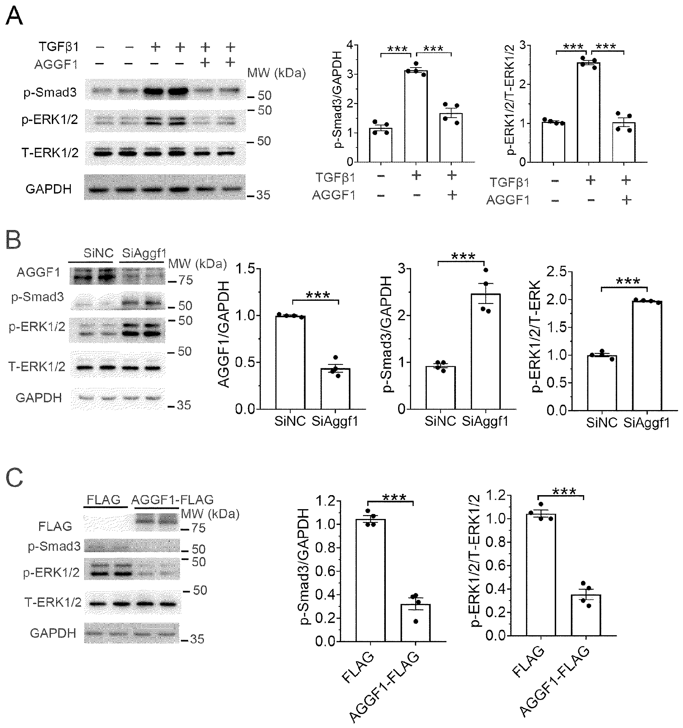

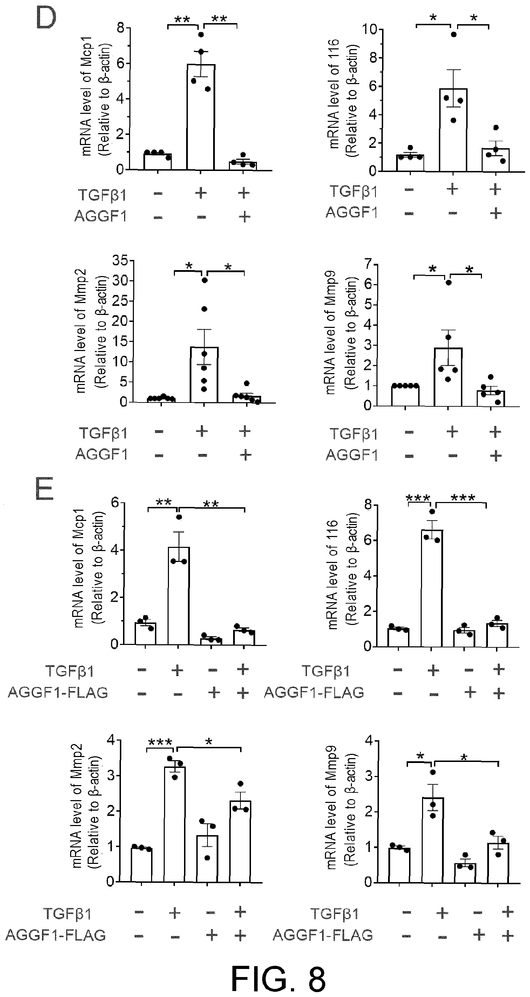

FIG. 8 depicts that AGGF 1 inhibits TGF-β1 and ERK1/2 signaling pathways and a TGF-β1-induced VSMC inflammation. FIG. (A) shows that VSMC cells are starved in advance for 12 hours, then treated with or without TGF-β1 for 15 minutes, followed by a AGGF1 treatment for 10 minutes. Subsequently, cell lysates are collected for the western blot analysis of p-ERK1/2, T-ERK1/2, and p-Smad3. The GAPDH serves as a loading control. The bar graph on the right displays quantification data. FIG. (B) shows that VSMC cells are transfected with a siRNA targeting Aggfl (SiAggfl) or a negative control siRNA (SiNC) to reduce an AGGF1 expression. After 48 hours, the cell lysates are collected for the western blot analysis of p-ERK1/2, T-ERK1/2, and p-Smad3. The bar graph on the right displays quantification data. FIG. (C) shows that the VSMC cells overexpressing AGGF1-FLAG or FLAG (control) are lysed after 48 hours for the western blot analysis of p-ERK1/2, T-ERK1/2, and p-Smad3. The GAPDH serves as a loading control. The bar graph on the right displays quantification data. FIG. (D) shows that VSMC cells are treated with TGF-β1 for 24 hours, followed by the AGGF1 treatment for 24 hours. Subsequently, mRNA is extracted using a Trizol method and reverse transcribed to obtain cDNA for a real-time RT-PCR detection of Il-6, Mcp 1, Mmp2, and Mmp9 mRNA levels. FIG. (E) shows that the VSMC cells overexpressing AGGF1-FLAG or FLAG (control) are stimulated with TGF-B1 for 48 hours after 24 hours of transfection. Subsequently, mRNA is extracted using the Trizol method and reverse transcribed to obtain cDNA. The real-time RT-PCR is performed to analyze the mRNAlevels of Il-6, Mcp1, Mmp2, and Mmp9. *, P<0.05; **, P<0.01; ***, P<0.001; n≧3/group.

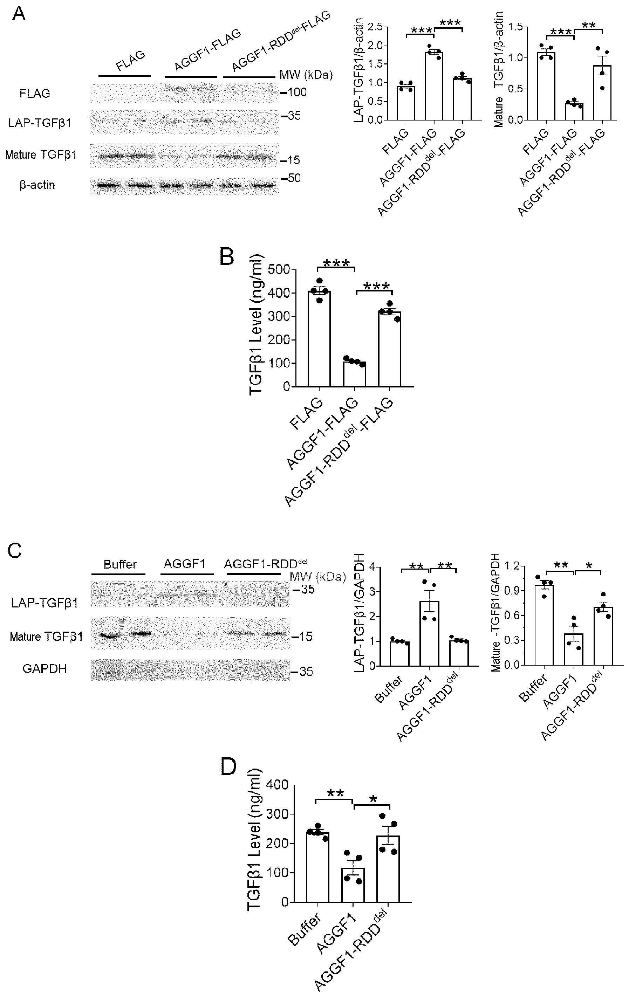

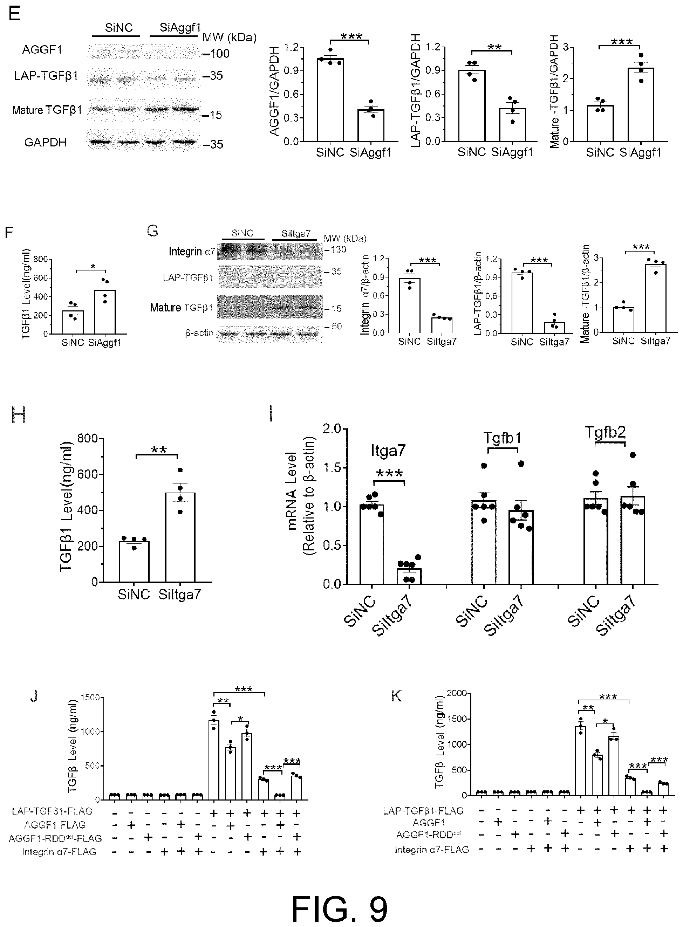

FIG. 9 demonstrates that the AGGF1 inhibits TGF-β1 maturation, whereas knockdown of Aggf1 or Itga7 induces TGF-β1 maturation. FIG. (A) shows a western blot analysis of LAP-TGF-B1 and the mature TGF-B1 in the VSMC cells overexpressing a wild-type AGGF1 or the mutant AGGF1-RDDdel after 48 hours. The β-actin serves as a loading control. FIG. (B) shows an ELISA measurement of TGF-β1 levels in a culture medium of the VSMC cells overexpressing AGGF1 or AGGF 1-RDDdel. FIG. (C) shows a Western blot analysis of an LAP-TGF-B1 and the mature TGF-B1 in the VSMC cells treated with AGGF1 or AGGF1-RDDdel for 48 hours. The GAPDH serves as a loading control. FIG. (D) shows the ELISA measurement of the TGF-β1 levels in the culture medium of the VSMC cells treated with AGGF1 or AGGF1-RDDdel for 48 hours. FIG.(E) shows the western blot analysis of the LAP-TGF-β1 and the mature TGF-B1 in the VSMC cells transfected with SiAggf1 or SiNC after 48 hours. FIG. (F) shows the ELISA measurement of the TGF-β1 levels in the culture medium of the VSMC cells transfected with SiAggfl or SiNC. FIG. (G) shows a western blot analysis of an integrin α7, LAP-TGF-β1, and the mature TGF-B1 in the VSMC cells transfected with SiItga7 or SiNC after 48 hours. FIG. (H) shows the ELISA measurement of the TGF-β1 levels in the culture medium of the VSMC cells transfected with SiItga7 or SiNC. FIG. (I) shows a real-time RT-PCR analysis of Itga7, Tgfb1, and Tgfb2 in the VSMC cells transfected with SiItga7 or SiNC after 48 hours. The mRNA is extracted using the Trizol method and reverse transcribed to obtain cDNA. FIG. (J) shows an ELISA measurement of the TGF-β1 levels in a culture medium of HEK293T cells. Transfected plasmids include LAP-TGF-β1-FLAG, the integrin α7-FLAG, AGGF1-FLAG, or AGGF1-RDDdel-FLAG, with grouping as shown in FIG. J. FIG. (K) shows an ELISA analysis of the TGF-β1 levels in the culture medium of the HEK293T cells. Cells are transfected with different plasmids of LAP- TGF -β1-FLAG and the integrin α7-FLAG, and treated with the purified AGGF1 or AGGF1-RDDdel for 24 hours, with grouping as shown in FIG. K. *, P<0.05; **, P<0.01; ***, P<0.001; n≥3/group.

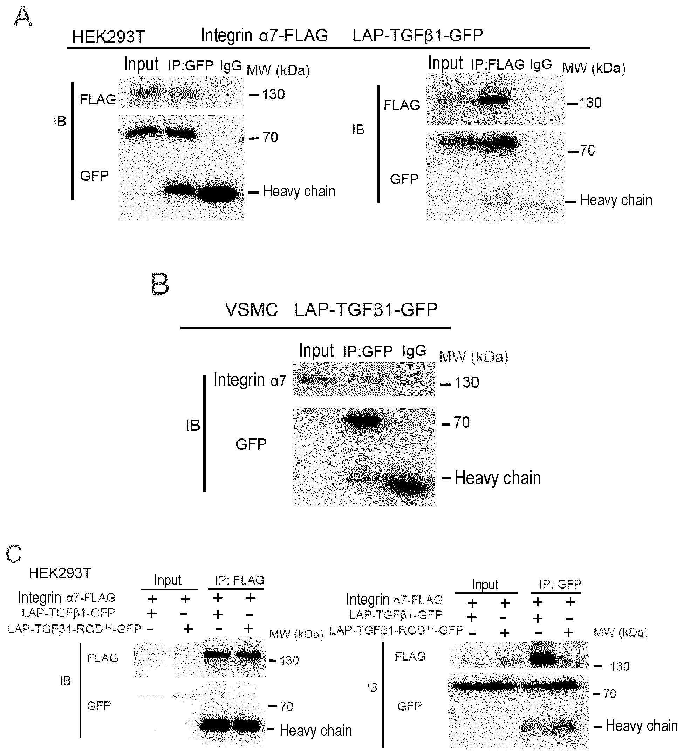

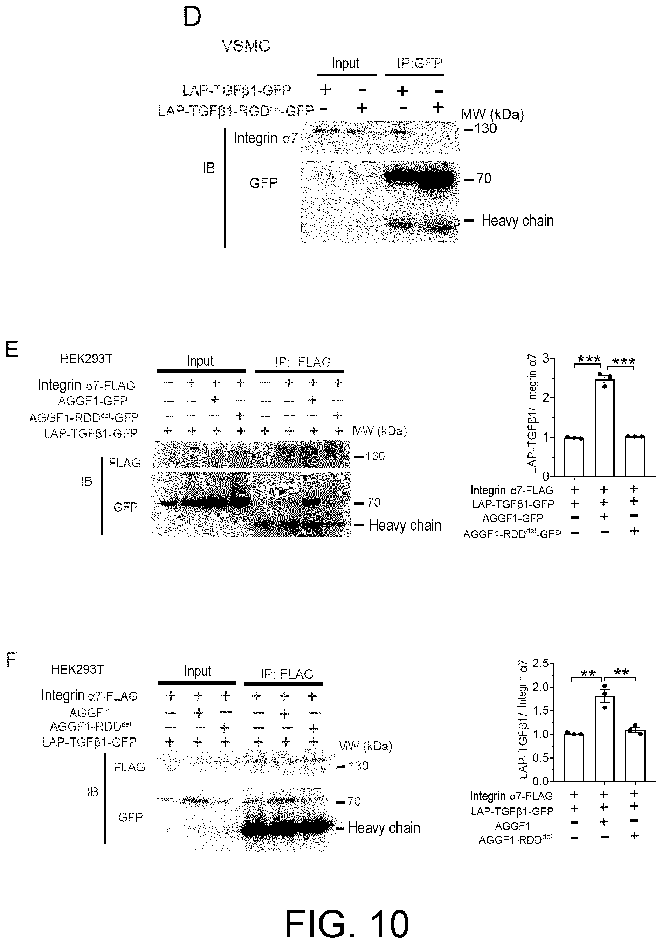

FIG. 10 illustrates that the integrin α7 interacts with LAP-TGF-β1 via an RGD sequence, and AGGF1 enhances this interaction. FIG. (A) shows a co-immunoprecipitation (Co-IP) analysis of the HEK293T cells overexpressing LAP-TGF-(31-GFP and the integrin α7-FLAG. FIG. (B) shows a Co-IP analysis of the VSMC cells overexpressing LAP-TGF-(31-GFP and an endogenous integrin α7. FIG. (C) shows a Co-IP analysis of the HEK293T cells overexpressing the wild-type LA₋P-TGF-β1-GFP or the mutant LA₋P-TGF-β1-RGDdel-GFP and the integrin α7-FLAG. FIG. (D) shows a Co-IP analysis of the VSMC cells overexpressing LAP-TGF-(31-GFP or LAP-TGF-β1-RGDdel-GFP. FIG. (E) shows a Co-IP analysis of the HEK293T cells overexpressing LAP-TGF-β1-GFP, α7-FLAG, the wild-type AGGF1-GFP, or the mutant AGGF 1-RDDdel-GFP. Quantitative data of a LAP-TGF-β1-GFP/α7-FLAG ratio are plotted as bar graphs on the right. FIG. (F) shows a Co-IP analysis of the HEK293T cells overexpressing LAP-TGF-β1-GFP and the integrin α7-FLAG, stimulated with AGGF1 or AGGF1-RDDdel for 24 hours. Quantitative data of the LAP-TGF-β1-GFP/α7-FLAG ratio are plotted as bar graphs on the right. *, P<0.05; **, P<0.01; ***, P<0.001; n = 3/group.

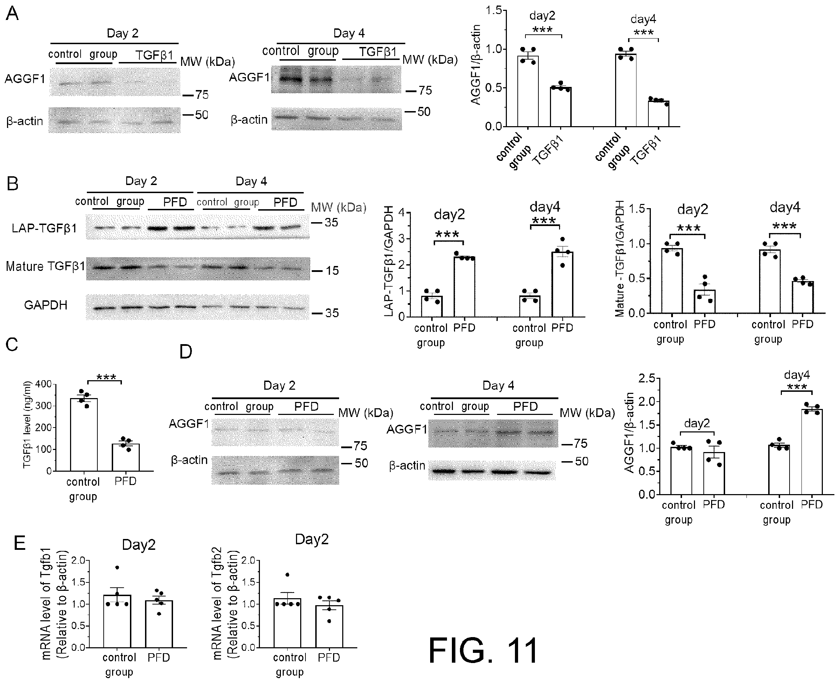

FIG. 11 demonstrates that TGF-β1 inhibits the AGGF1 expression, while pirfenidone (PFD) increases the AGGF1 expression. FIG. (A) shows a western blot analysis of AGGF1 in the VSMC cells after treatment with TGF-β1 for 2 days and 4 days. The VSMCs are starved for 12 hours prior to stimulation with TGF-β1. FIG. (B) shows a western blot analysis of LAP-TGF-β1 and the mature TGF-β1 in the VSMC cells after treatment with PFD for 2 days and 4 days. FIG. (C) shows an ELISA measurement of the mature TGF-β1 levels in a culture medium from the VSMCs treated with PFD for 2 days. FIG. (D) shows the western blot analysis of AGGF1 in the VSMCs treated with PFD for 2 days and 4 days. FIG. (E) shows a real-time RT-PCR analysis to detect mRNA levels of Tgfb1 and Tgfb2 from the VSMCs treated with PFD for 2 days. Normalized to β-actin. *, P<0.05; **, P<0.01; ***, P<0.001; n≥4/group for the study.

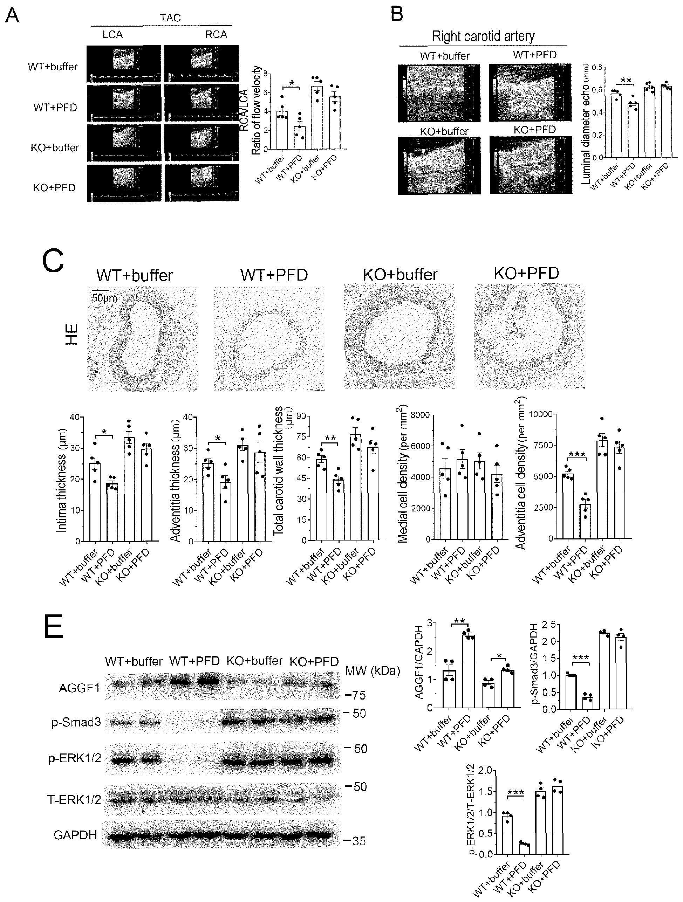

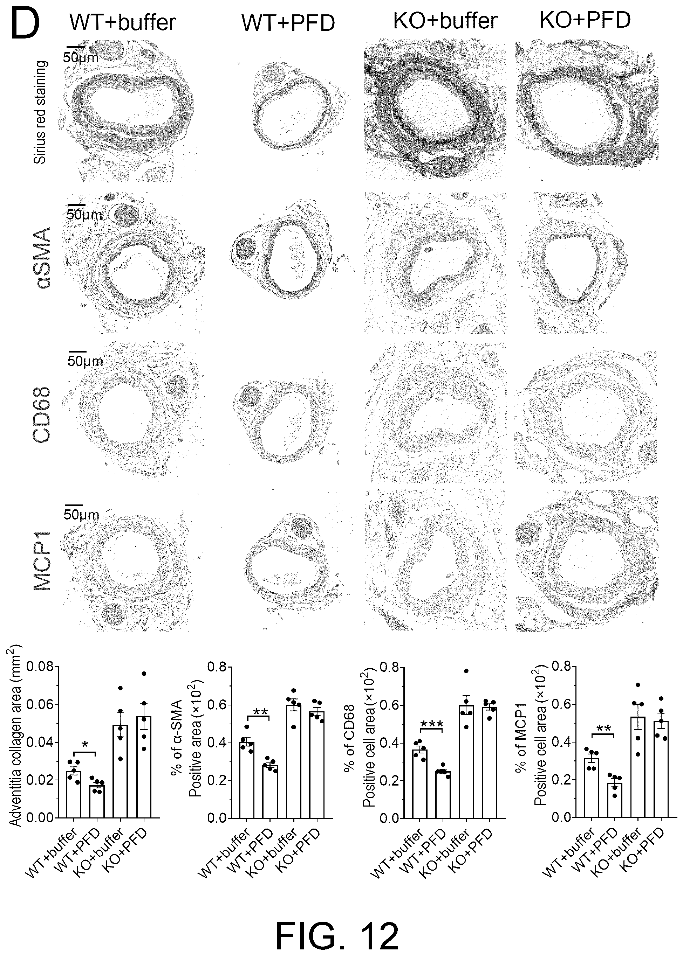

FIG. 12 demonstrates that pirfenidone attenuates the TAC-induced carotid artery dilation, remodeling, and inflammation by inhibiting Smad3 and ERK1/2 phosphorylation in Aggfl^{+/+} (WT) mice, but not in Aggfl^{+/-} (KO) mice. FIG. (A) shows echocardiographic images of the LCA and the RCA in WT or KO mice 3 weeks after TAC. The bar graph on the right shows the ratio of the flow velocity (FV) between the RCA and the LCA. FIG. (B) shows a mean luminal diameter of the RCA 3 weeks after TAC. FIG. (C) shows the H&E staining of the cross-sections of the RCA. The bar graphs below show the intimal thickness (1st graph), the adventitia thickness (2nd graph), the total carotid artery wall thickness (3rd graph), the medial cell density (4th graph), and the adventitia cell density (5th graph). FIG. (D) shows the Sirius red staining and the immunostaining for α-SMA, MCP-1, and CD68 in the cross-sections of the RCA. The bar graphs on the right show the percentage of the positive area for the respective immunostaining quantification. FIG. (E) shows an immunoblot analysis ofAGGF1, p-ERK1/2, p-Smad3, and T-ERK1/2 in the RCA. The GAPDH serves as a loading control. *, P<0.05; **, P<0.01; ***, P<0.001; n=5/group for mouse studies, other sample sizes n=4/group.

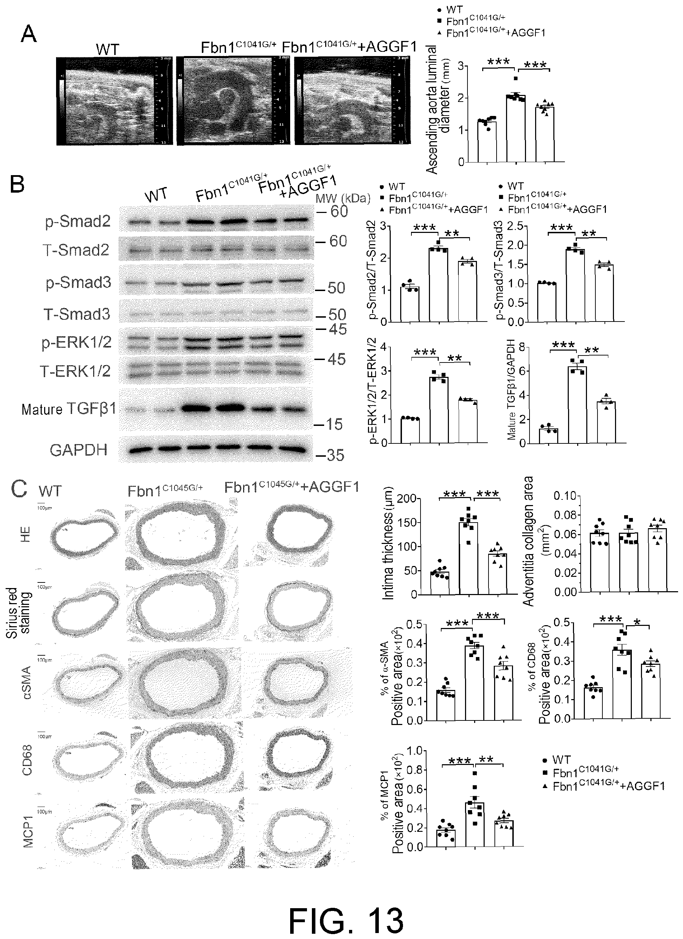

FIG. 13 depicts an AGGF1 treatment for a spontaneous aortic aneurysm in FbnC1041G/+ mutant mice. FIG. (A) shows echocardiographic images of the aorta from the wild-type (WT) or FbnC1041G/+ mutant male mice at week 18. The bar graph on the right illustrates the luminal diameter of the ascending aortic. FIG. (B) shows an immunoblot analysis of p-Smad2, p-Smad3, p-ERK, and the mature TGFβ1 in the ascending aorta of WT or FbnC1041G/+ mutant male mice. The GAPDH serves as a loading control. FIG. (C) shows the Sirius red staining and the immunostaining for α-SMA, MCP-1, and CD68 in the cross-sections of the ascending aorta. The bar graphs on the right indicate the percentage of the positive area for the respective immunostaining quantification. *, P<0.05; **, P<0.01; ***, P<0.001; n=8/group for mouse studies, other sample sizes n=4/group.

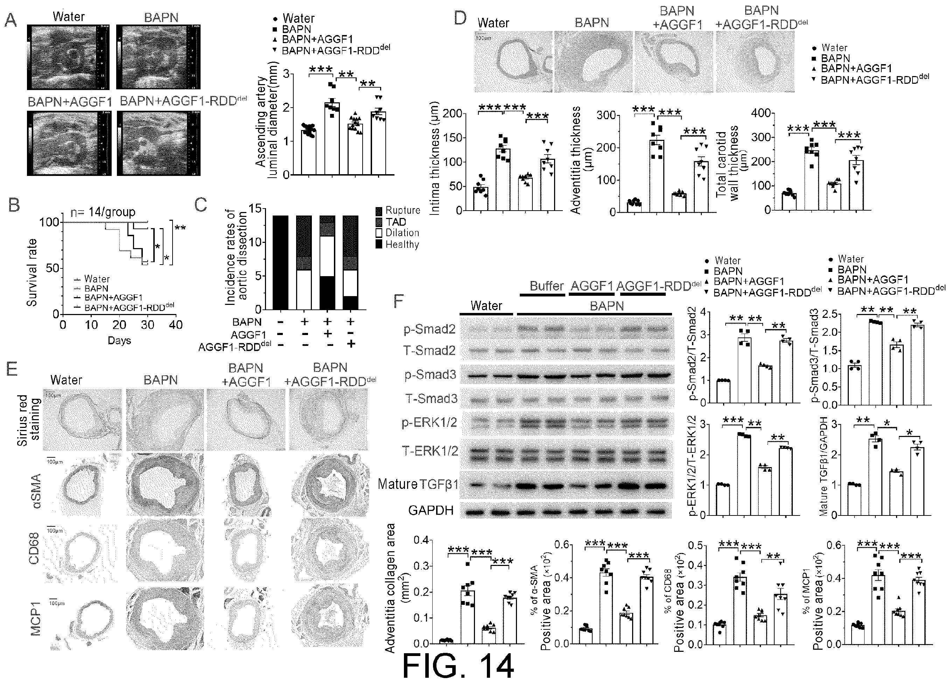

FIG. 14 depicts an AGGF1 treatment for a β-aminopropionitrile (BAPN)-induced aortic aneurysm. FIG. (A) shows echocardiographic images of the aorta in 3-week-old mice after 28 days of water or BAPN feeding. The bar graph on the right shows the luminal diameter of the ascending aorta. FIG. (B) shows survival rates of the four groups of mice in the induced aortic aneurysm model. FIG. (C) shows incidence rates of aortic dissection in the four groups of mice in the induced aneurysm model. FIG. (D) shows the H&E staining of the cross-sections of the ascending aorta. The bar graphs below show the intimal thickness (1st image), the adventitia thickness (2nd image), and the total carotid artery wall thickness (3rd image). FIG. (E) shows the Sirius red staining and the immunostaining for α-SMA, MCP-1, and CD68 in the cross-sections of the ascending aorta. The bar graphs on the right show the percentage of the positive area for respective immunostaining quantifications. FIG. (F) shows a western blot analysis of p-Smad2, p-Smad3, p-ERK, and the mature TGFβ1 in the ascending aorta of the four groups of model mice. The GAPDH serves as a loading control. *, P<0.05; **, P<0.01; ***, P<0.001; n≥8/group for mouse studies, other sample sizes n=4/group.

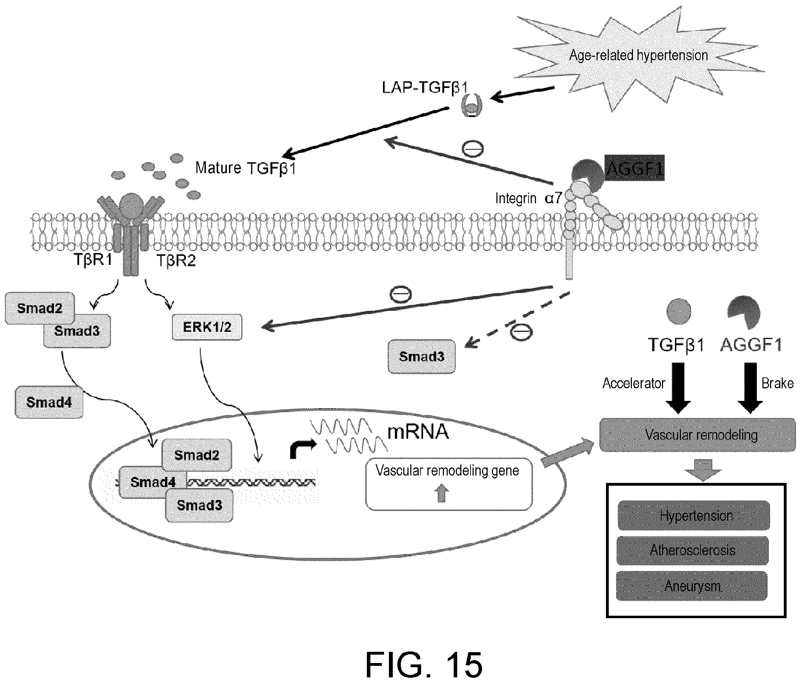

FIG. 15 illustrates a mechanism by which AGGF1 acts on the smooth muscle cells through the integrin α7. Diseases such as hypertension and vascular aging may stimulate cells to synthesize TGFβ1, which is secreted and cleaved extracellularly into the mature TGFβ1. The mature TGFβ1 binds to dimers of type II receptors (TβRII) on the cell membrane, which then recruit type I receptor dimers (TβRI) to form a heterotetrameric complex. The type I receptor is a serine/threonine kinase receptor that stimulates the phosphorylation of Smad2/3 and ERK1/2. Phosphorylated Smad2/3 recruits Smad4, forming a complex that translocates to the nucleus to regulate target gene expression, while ERK1/2 may directly enter the nucleus to regulate gene expression. These genes promote vascular remodeling, ultimately leading to diseases such as an aneurysm and an atherosclerosis. AGGF1 functions in two ways: it promotes the binding of the integrin α7 to LAP-TGFβ1, thereby inhibiting a cleavage thereof, and suppresses the phosphorylation of Smad2/3 and ERK1/2, blocking the TGFβ1 signaling pathway. In the pathogenesis of diseases such as an aneurysm, TGFβ1 acts as an "accelerator," while AGGF1 serves as a "brake" against TGFβ1.

### [DESCRIPTION OF THE EMBODIMENTS]

In order to make the purpose, technical solution and advantages of the present disclosure more clear, the following further detailed description of the present disclosure is provided in conjunction with the drawings and embodiments.

It should be understood that the specific embodiments described here are only used to explain the present disclosure, and are not used to restrict the present disclosure. In addition, the technical features involved in the various embodiments of the present disclosure described below may be combined with each other as long as they do not conflict with each other. Without departing from the above technical ideas and disclosed spirit of the present disclosure, according to the ordinary technical knowledge and conventional means in the field, various equivalents, substitutions, modifications or changes made all fall within the scope to be protected by the present disclosure.

In order to effectively control the occurrence of an aneurysm and clinical interventions, to explore the pathogenesis of the aneurysm and find effective intervention methods, the present disclosure provides an in vitro purified recombinant protein, namely an AGGF1 protein, for preparing anti-aneurysm drugs, which has the effect of treating an aortic aneurysm and a carotid artery aneurysm. The present disclosure provides a recombinant protein, a nucleic acid sequence encoding the recombinant protein is: a protein encoded by a nucleic acid sequence shown in SEQ ID NO.2.

The recombinant protein is named the AGGF1 protein, and an encoding gene thereof was discovered and cloned through a gene linkage positioning analysis in a Klippel-Trenaunay syndrome (abbreviated as KTS). The AGGF1 protein has extremely high expression levels in endothelial cells, smooth muscle cells and osteoblasts.

In the present disclosure, the cloning method of the recombinant protein gene that inhibits the aneurysm uses a forward primer shown in SEQ ID NO:3 and a reverse primer shown in SEQ ID NO:4, and a PCR method is applied to clone the AGGF1 gene from human cDNA.

In the present disclosure, a recombinant plasmid expressing the recombinant protein gene that inhibits the aneurysm. The AGGF1 gene is connected with a His tag sequence, and inserted into a multiple cloning restriction site of an expression vector pet28a to obtain a pet28a-AGGF1 plasmid, which is the recombinant plasmid.

In the present disclosure, a recombinant protein that inhibits the aneurysm, and the amino acid sequence of the protein is as follows:
(1) As shown by an amino acid sequence of SEQ ID NO: 1; or
(2) An amino acid sequence having 80% to 100% homology with the amino acid sequence defined by SEQ ID NO: 1, and retaining the amino acids at positions 606-608, wherein the amino acid sequence encodes a protein with the same function; or
(3) An amino acid sequence defined by SEQ ID NO: 1 that has undergone addition, deletion, or substitution of one or more amino acids, and that retains the amino acids at positions 606-608, possessing an equivalent activity to the protein shown in the sequence of SEQ ID NO: 1.

A preparation method for a recombinant protein that inhibits the aneurysm in the present disclosure includes the following steps:
(1) After a human source AGGF1 gene is connected with a His tag sequence, insertion of the same is made into a multiple cloning restriction site of a prokaryotic expression vector pet28a, thus constructing a recombinant plasmid pet28a-AGGF1 containing the AGGF1 gene.
(2) The recombinant plasmid described in step (1) is transfected into Escherichia coli BL21(DE3), and screened through a kanamycin resistance to obtain positive cell clones with an antibiotic resistance.
(3) After the positive cell clones obtained in step (2) are expanded and cultured, the same are isolated and purified to obtain the recombinant protein.

### Example 1

Preparation of AGGF1 recombinant protein: The AGGF1 protein expression plasmid pet28-AGGF1 was expressed in Escherichia coli BL21(DE3). The expression strain was cultured overnight in 20ml of a LB medium with kanamycin resistance. The next day, this 20 ml culture was added to 1 L of the LB medium with kanamycin resistance and cultured in a shaker at 37°C for 2.5 hours. Then 1 mM IPTG was added to induce protein expression. IPTG is a highly stable lactose analog that may inhibit the lac repressor protein and induce the synthesis of β-galactosidase. This enzyme promotes the utilization of lactose. IPTG may be used to induce the expression of target genes regulated by the lac operon. After adding IPTG for induction, the E. coli continued to grow in the medium for 5.5 hours. The medium was then centrifuged at 4000 rpm, 4°C, for 10mins. If the protein was not immediately isolated, the E. coli precipitate was stored at -20°C. The purification of recombinant protein utilized the QIAexpressionist high-level expression and six histidine-tagged protein purification manual (Qiagen). 6ml buffer (50 mM NaH₂PO₄, 300 mM NaCl, 20 mM imidazole, 0.05% Tween-20, pH8.0) was prepared with the addition of protease inhibitor mixture (6 µg/ml chymostatin, 1 µg/ml E64, 2 µg/ml aprotinin, 0.5 µg/ml phosphoramidon, 1 µg/ml pepstatin A, 5 µg/ml leupeptin, 5 µg/ml antipain, 0.1 mM benzamidine (Sigma)). The buffer was added to the E. coli precipitate after centrifugation of 1 L medium, the bacteria were resuspended, and 1 mg/ml lysozyme (Invitrogen) was added to the suspension, followed by an ice bath for 30 minutes. The suspension was disrupted using an ultrasonic probe of an ultrasonic disruptor. The power was 300W, with each sonication time of 10s, gap time of 10s, performed 6 times. The disrupted product was transferred to EP tubes and centrifuged at 12000 rpm, 4°C, for 50 mins. The supernatant was collected and kept on ice. Ni-NTA agarose beads were used to purify his-tagged proteins from the supernatant. Ni-NTA metal chelation affinity chromatography has very high affinity for biomolecules with hexahistidine tags. Ni-NTA beads were washed twice with 5ml of EB, and the supernatant was added to the beads (1 ml supernatant per portion of beads), and the mixture was incubated overnight in a shaker at 4°C. The next day, the supernatant was discarded after centrifugation. The beads were washed three times with buffer (50 mM NaH₂PO₄, 300 mM NaCl, 20 mM imidazole, 0.05% Tween-20, pH8.0) to reduce non-specific binding proteins. The 6X-his tagged protein was eluted with 1ml elution buffer (50 mM NaH₂PO₄, 300 mM NaCl, 250 mM imidazole, 0.05% Tween-20, pH 8.0). The eluted protein was dialyzed twice using Spectra/Por dialysis tubing (Spectrum Laboratories, Inc., USA) in 1 X EB at 4°C. After dialysis, the purified protein was aliquoted and stored at -80°C, which is the recombinant protein provided by the present disclosure.

### Example 2

Preparation of AGGF1-RDD^{del} protein: A protein with deletion of three amino acids (positions 606-608, sequence RDD) in the AGGF1 recombinant protein amino acid sequence (a homologous protein obtained through the truncation of the AGGF1 protein sequence) is prepared. Using eukaryotic expression pcDNA3.1-AGGF1 as a template, a DNA fragment of the AGGF1 gene with 9 base pairs deleted was amplified by PCR. The obtained fragment was cloned into the pet28 protein expression plasmid after double enzyme digestion. The plasmid was transformed into Ecoli BL21, and IPTG was used to induce the expression of the AGGF1 gene truncated fusion protein. Western blotting was used to identify the expression of the AGGF1 gene truncated fusion protein. The preparation method was the same as in Example 1.

### Example 3

Characterization changes in TAC surgery-induced aneurysm model in Aggf1 knockout mice.

In this experiment, two strains of mice were used: one strain of mice were Aggfl systemic knockout mice (Aggfl ^{+/-}), with wild-type mice (WT) as the control mice (see FIG. 1); the other strain of mice were Aggfl smooth muscle-specific knockout mice (Aggfl^{smcKO}), with mice carrying only loxp sites as control mice (Aggfl^{fl/fl},CTL) (see FIG. 2). Each group of mice was divided into sham and TAC groups according to whether TAC surgery was performed, with a mouse sample size ≥5. 21 days after the TAC surgery, Doppler ultrasound was used to detect the vascular luminal diameter, and carotid artery and aorta were collected for histological staining and Western blot to evaluate the effect of AGGF1. As shown in FIG. 1 and FIG. 2, in Aggfl^{+/-}mice and Aggfl^{smcKO}mice, there was no difference in carotid artery luminal diameter and histological staining in the sham group, but after TAC surgery, compared with their control groups, both Aggfl^{+/-} mice and Aggfl^{smcKO}mice showed significant vascular expansion (blood flow velocity and vascular luminal diameter), remodeling (H&E staining, Sirius red staining and αSMA) and inflammation (MCP1 and CD68). Western blot assessment showed that after TAC surgery, the level of AGGF1 in the carotid artery of both strains of mice decreased, while p-Smad3 and p-ERK1/2 were significantly increased. Moreover, the level of mature TGFβ1 in Aggfl^{smcKO}mice was also significantly increased as shown in FIG. 2. These results highlight the important role of AGGF1 in blood vessels.

Use of recombinant proteins synthesized in Examples 1 and 2 in the preparation of anti-aneurysm drugs.

The research team obtained blood vessel samples from the thoracic aorta of 8 normal individuals and 8 patients with the thoracic aortic aneurysm from the hospital. As shown in FIG. 3A, AGGF1 immunostaining showed that the level of AGGF1 was significantly reduced in aneurysm samples. Meanwhile, consistent changes were also observed in the TAC mice; that is, AGGF1 protein expression was significantly reduced in both the arterial aneurysm mouse model and vascular tissues of the patients with the thoracic aortic aneurysm. Subsequently, the research divided C57BL/6 mice into 6 groups: after sham surgery, they were divided into a buffer group, an AGGF1 group, and an AGGF1-RDD^{del} group (Sham +buffer, Sham +AGGF 1, Sham +AGGF 1-RDD^{del}), and after the TAC surgery, they were divided into a buffer group, an AGGF1 group, and an AGGF1-RDD^{del} group (TAC+buffer, TAC+AGGF1, TAC+AGGF1-RDD^{del}), with each group having mice ≥6. Buffer was protein elution solution. 21 days after the TAC surgery, Doppler ultrasound was used to detect vascular luminal diameter, and carotid artery and aorta were collected for histological staining and Western blot to evaluate the effect of AGGF1. Starting from the second day after the surgery, recombinant AGGF1 protein or AGGF1-RDD^{del} protein was administered through intraperitoneal injection in the mice, with the same dose of EB as control, once every 2 days for 3 weeks. The protein dosage was 0.5 mg/kg.

As shown in FIG. 3, in the sham group, AGGF1 protein or AGGF1-RDD^{del} protein had little effect on the carotid artery and aorta of mice, but after the TAC surgery, compared with control buffer-treated mice with TAC-induced aneurysm, AGGF1 protein could significantly inhibit vascular expansion and remodeling, while AGGF1-RDD^{del} protein did not have these effects. In FIG. 4, compared with control buffer-treated mice with the TAC-induced aneurysm, AGGF1 protein could significantly inhibit vascular inflammation, and regardless of sham or TAC surgery, AGGF1 protein could inhibit p-Smad3, p-ERK1/2, and the level of the mature TGFβ1. However, AGGF1-RDD^{del} protein did not have these effects. In FIG. 5, the expansion of the ascending aorta and vascular thickening (H&E staining) in mice were detected. As the results show, AGGF1 protein could significantly inhibit ascending aortic expansion and vascular thickening. FIG. 6 shows that after the TAC surgery, remodeling and inflammation of the ascending aorta were significantly enhanced and were inhibited by AGGF1 protein, while in FIG. 7, Western blot analysis shows that AGGF1 protein could inhibit p-Smad3, p-ERK1/2, and the level of the mature TGFβ1. AGGF1-RDDdel protein did not have these effects.

These data indicate that AGGF1 protein therapy successfully reduced vascular lesions in the TAC-induced mouse aneurysm model through inhibiting vascular expansion, remodeling, and inflammation.

In vitro cellular experimentation was conducted to investigate the molecular mechanisms by which AGGF1 counteracts aneurysm formation.

In vitro experiments were performed using aortic smooth muscle cells and HEK293T cells. As shown in A-C of FIG. 8 and A-F of FIG. 9, whether with recombinant AGGF1 protein, AGGF1-FLAG overexpression, or AGGF1 silencing, Western blot analysis and ELISA experimental results verified that AGGF1 inhibits the TGFβ1 signaling pathway (Smad3 and ERK1/2) and the level of the mature TGFβ1. In FIG. D to FIG. E of FIG. 8, RT-PCR results show that recombinant AGGF1 protein and AGGF1-FLAG overexpression inhibit TGFβ1-induced inflammation (Il6, Mcp1, Mmp2, Mmp9). In FIG. G to FIG. H of FIG. 9, Western blot analysis and ELISA results show that after silencing integrin α7, the level of mature TGFβ1 was significantly increased. The RT-PCR results in FIG. I of FIG. 9 show that silencing integrin α7 had no effect on the mRNA level of TGFβ1. The ELISA experimental results in FIG. J to FIG. K of FIG. 9 show that AGGF1-FLAG overexpression or recombinant AGGF1 protein could enhance the inhibition of mature TGFβ1 by integrin α7. However, in these experiments, AGGF1-RDD^{deI} protein or AGGF1-RDD^{del}-FLAG did not have these effects. In the Western blot analysis of FIG. 10, it was verified that LAP-TGFβ1 interacts with integrin α7 through the RGD sequence, which is also the molecular mechanism by which integrin α7 inhibits TGFβ1.

AGGF1 is an important mediating factor for pirfenidone in treating the aneurysm.

In FIG. 11, we used VSMC in vitro incubated with TGF-β1 and pirfenidone (PFD). FIG. A shows that through Western experiment, TGF-β1 was found to inhibit the level of AGGF 1, while in FIG. B to FIG. D, PFD not only inhibited the synthesis of the mature TGF-β1, but also increased the level of AGGF1. However, in FIG. E, RT-PCR results show that PFD had no effect on the mRNA levels of Tgfb1 and Tgfb2 after 2 days. In FIG. 12, we used Aggfl systemic knockout mice for experiments. In FIG. A to FIG. B of FIG. 12, PFD could significantly reduce the expansion of the right carotid artery and the ratio of the flow velocity between right/left carotid aneurysm blood in the WT mice. In the staining of FIG. C to FIG. D, it can also be seen that PFD could significantly reduce the right carotid artery vascular remodeling (H&E staining, Sirius red and αSMA) and inflammation (MCP1 and CD68) in the WT mice. However, PFD did not possess such effects in Aggf1 heterozygous knockout mice (Aggfl^{+/-}; Aggf1 expression reduced by 1/2). The Western blot experiments show that PFD could significantly increase the level of AGGF1 in the RCA of the WT mice after TAC, and reduce the levels of p-Smad3 and p-ERK1/2, but did not have this effect in Aggfl ^{+/-} mice. Through this part of the results, we found that TGF-β inhibitors may increase AGGF1 expression levels. In the meantime, from the molecular mechanism study, it was found that maintaining relatively high levels of AGGF1 is necessary for TGF-β inhibitors to exert therapeutic effects.

AGGF1 protein inhibits the aortic disease of mice induced by FbnI^{C1041G/+} mutation.

The present disclosure obtained the previously published Fbnl^{C1041G/+} mutant mice from Tongji Hospital. Compared with the wild-type mice, we detected spontaneous aortic expansion in these mice at week 10, resulting in the aortic aneurysm. After obtaining these mice, at week 10, mice were intraperitoneally injected with Buffer or AGGF1 protein, and the injection continued until week 18. At week 18, mice underwent ultrasound examination, then were euthanized and their aortas were collected for Western Blotting experiments and histochemical staining. In FIG. 13, as shown in FIG. 13A, at week 18, compared with the wild-type mice, the ascending aorta of Fbnl^{C1041G} mutant mice expanded by more than 50%, while AGGF1 protein could significantly inhibit this expansion. Meanwhile, the Western blot results in FIG. 13B show that p-Smad2, p-Smad3, p-ERK and the level of mature TGFβ1 were significantly increased in the Fbnl^{C1041G/+} mutant mice, and all could be inhibited by the AGGF1 protein. The immunohistochemistry results in FIG. 13C show that the aortic media of the Fbn1^{C1041G/+} mutant mice was significantly thickened, with no change in the adventitia, while the positive areas of αSMA/CD68/MCP1 immunostaining were also significantly increased. The AGGF1 protein could significantly inhibit the thickening of the vascular media and the increase in αSMA/CD68/MCP1 levels.

The AGGF1 protein inhibits the aortic aneurysm of mice induced by β-aminopropionitrile (BAPN).

β-aminopropionitrile is a lysyl oxidase (LOX) inhibitor that is readily soluble in water. Three-week-old mice fed with 0.25% BAPN have a very high incidence of the thoracic aortic aneurysm after 4 weeks. Three-week-old C57BL/6 male mice were divided into 4 groups, with ultrasound detection of the aorta on day 28, followed by euthanasia and collection of the aorta for Western Blotting experiments and histochemical staining. In FIG. 14, as shown in FIG. 14A, ultrasound results show that compared with the control group, BAPN significantly induced ascending aortic expansion, AGGF1 possessed an inhibitory effect on vascular expansion, while AGGF1-RDDdel protein lacked this effect. In FIG. 14B, the survival rate of mice in the BAPN group was only 57%, while the AGGF1 protein could significantly improve the survival rate of these aneurysm model mice, and AGGF1-RDD^{del} did not possess this effect. FIG. 14C shows that AGGF1 had no effect on the incidence of BAPN-induced aortic dissection. In the H&E staining in FIG. 14D, the media and adventitia of the aorta in the BAPN group were significantly thickened. In the immunohistochemistry in FIG. 14E, the positive areas of αSMA/CD68/MCP1 in the aorta of the BAPN group were significantly increased. AGGF1 significantly inhibited aortic thickening, remodeling and inflammation, while the AGGF1 protein with deleted RDD sequence showed significantly reduced effects. The Western blot results in FIG. 14F show that p-Smad2, p-Smad3, p-ERK and the level of the mature TGFβ1 were significantly increased in mice of the BAPN group, and all could be inhibited by the AGGF1 protein, while AGGF1-RDDdel protein did not possess this effect.

In summary, the present disclosure employed three aortic aneurysm models: first, an aortic aneurysm-related arterial remodeling model was established through transverse aortic constriction (TAC); second, a Marfan syndrome-thoracic aortic aneurysm model induced by Fbnl^{C1041G/+} mutation was established; and third, a thoracic aortic aneurysm model induced by BAPN was established. All three models verify that the AGGF1 protein treatment may successfully inhibit vascular inflammation and remodeling associated with the aortic aneurysm.

FIG. 15 is a schematic diagram showing the mechanism by which AGGF1 acts on smooth muscle cells through its receptor integrin α7. Diseases such as hypertension and vascular aging may stimulate cells to synthesize TGF-B1 and secrete it extracellularly where it is cleaved into mature TGF-β1. The mature TGF-β1 binds to type II receptor dimers (TβRII) on the cell membrane, and type II receptors recruit type I receptor dimers (TβRI) to form a heterotetrameric complex. The type I receptor is a serine/threonine kinase receptor that may stimulate Smad2/3 and ERK1/2 phosphorylation, where Smad2/3 recruits and binds with Smad4 to enter the nucleus, thereby regulating the expression of target genes, while ERK1/2 may directly enter the nucleus to regulate gene expression. These genes may promote vascular remodeling, ultimately leading to diseases such as an aneurysm and an atherosclerosis. On one hand, AGGF1 may promote the binding of integrin α7 and LAP- TGFβ1, thereby inhibiting its cleavage; on the other hand, it may inhibit Smad2/3 and ERK1/2 phosphorylation, thus blocking the TGFβ1 signaling pathway.

AGGF1 interacts with its receptor integrin α7, enhancing the interaction between integrin α7 and LAP-TGF-β1, blocking the maturation of TGF-β1, thereby inhibiting TGF-B1 signaling pathway and ERK1/2 signaling pathway. Meanwhile, the combined application of AGGF1 and pirfenidone against the aneurysm is also quite necessary. The recombinant AGGF1 protein with His tag in the present disclosure may alleviate vascular lesions by inhibiting TGFβ1 synthesis and its signaling pathway, improve vascular function in mice, and ultimately inhibit the aneurysm. Therefore, the recombinant AGGF1 protein with His tag in the present disclosure may be prepared as an anti-aneurysm drug for the treatment of aneurysm-related diseases.

Those skilled in the art may easily understand that the above description is merely a preferred embodiment of the present disclosure and is not intended to restrict the present disclosure. Any modifications, equivalent substitutions, and improvements made within the spirit and principles of the present disclosure should be included within the protection scope of the present disclosure.

### Sequence Listing

Sequence Listing Information:
   DTD Version: V1_3
   File Name: Use of Recombinant Protein in Preparing Drug for Inhibiting Aneurysm.xml
   Software Name: WIPO Sequence
   Software Version: 2.1.0
   Generation Date: 2022-09-13
Basic Information:
   Current Application / Applicant File Name: Huazhong University of Science and Technology
   Applicant Name: Huazhong University of Science and Technology
   Applicant Name / Language: zh
   Applicant Name / Latin Name: Huazhong University of Science and Technology
   Title of Disclosure: Use of Recombinant Protein in Preparing Drug for Inhibiting Aneurysm (zh)
   Total Sequences: 4
Sequences:
   Sequence Number (ID): 1
      Length: 714
      Molecule Type: AA
      Characteristic Location/Qualifier:
         - source, 1..714
            > mol_type, protein
            > organism, synthetic construct
      Residues: 
MASEAPSPPR SPPPPTSPEP ELAQLRRKVE KLERELRSCK RQVREIEKLL
HHTERLYQNA 60
ESNNQELRTQ VEELSKILQR GRNEDNKKSD VEVQTENHAP WSISDYFYQT
 
YYNDVSLPNK 120
VTELSDQQDQ AIETSILNSK DHLQVENDAY PGTDRTENVK YRQVDHFASN
SQEPASALAT 180
EDTSLEGSSL AESLRAAAEA AVSQTGFSYD ENTGLYFDHS TGFYYDSENQ
LYYDPSTGIY 240
YYCDVESGRY QFHSRVDLQP YPTSSTKQSK DKKLKKKRKD PDSSATNEEK
DLNSEDQKAF 300
SVEHTSCNEE ENFANMKKKA KIGIHHKNSP PKVTVPTSGN TIESPLHENI
SNSTSFKDEK 360
IMETDSEPEE GEITDSQTED SYDEAITSEG NVTAEDSEDE DEDKIWPPCI
RVIVIRSPVL 420
QIGSLFIITA VNPATIGREK DMEHTLRIPE VGVSKFHAEI YFDHDLQSYV
LVDQGSQNGT 480
IVNGKQILQP KTKCDPYVLE HGDEVKIGET VLSFHIHPGS DTCDGCEPGQ
VRAHLRLDKK 540
DESFVGPTLS KEEKELERRK ELKKIRVKYG LQNTEYEDEK TLKNPKYKDR
AGKRREQVGS 600
EGTFQRDDAP ASVHSEITDS NKGRKMLEKM GWKKGEGLGK DGGGMKTPIQ
LQLRRTHAGL 660
GTGKPSSFED VHLLQNKNKK NWDKARERFT ENFPETKPQK DDPGTMPWVK
GTLE 714
   Sequence Number (ID): 2
      Length: 2145
      Molecular Type: DNA
      Characteristic Location/Qualifier:
         - source, 1..2145
            > mol_type, other DNA
            > organism, synthetic construct
      Residues: 
atggcctcgg aggcgccgtc cccgccgcgg tcgccgccgc cgcccacctc ccccgagcct 60
gagctggccc agctaaggcg gaaggtggag aagttggaac gtgaactgcg gagctgcaag 120
cggcaggtgc gggagatcga gaagctgctg catcacacag aacggctgta ccagaacgca 180
gaaagcaaca accaggagct ccgcacgcag gtggaagaac tcagtaaaat actccaacgt 240
gggagaaatg aagataataa aaagtctgat gtagaagtac aaacagagaa ccatgctcct 300
tggtcaatct cagattattt ttatcagacg tactacaatg acgttagtct tccaaataaa 360
gtgactgaac tgtcagatca acaagatcaa gctatcgaaa cttctatttt gaattctaaa 420
gaccatttac aagtagaaaa tgatgcttac cctggtaccg atagaacaga aaatgttaaa 480
tatagacaag tggaccattt tgcctcaaat tcacaggagc cagcatctgc attagcaaca 540
gaagatacct ccttagaagg ctcatcatta gctgaaagtt tgagagctgc agcagaagcg 600
gctgtatcac agactggatt tagttatgat gaaaatactg gactgtattt tgaccacagc 660
actggtttct attatgattc tgaaaatcaa ctctattatg atccttccac tggaatttat 720
tactattgtg atgtggaaag tggtcgttat cagtttcatt ctcgagtaga tttgcaacct 780
tatccgactt ctagcacaaa acaaagtaaa gataaaaaat tgaagaagaa aagaaaagat 840
ccagattctt ctgcaacaaa tgaggaaaag gatttgaact cagaggatca aaaagccttc 900
agtgttgaac atacaagctg caatgaggaa gaaaatttcg caaatatgaa aaagaaggcc 960
aaaataggca ttcatcacaa aaatagtccc cccaaagtca ctgttccaac tagtggaaat 1020
actatagagt ctcctcttca tgaaaacatc tctaattcaa catcatttaa agatgagaaa 1080
atcatggaga ctgatagtga accagaggaa ggtgaaatta cagactctca gactgaggat 1140
agttatgacg aagccattac cagtgaaggc aatgtaactg cagaagatag tgaggatgaa 1200
gatgaagaca aaatttggcc cccatgtatt agagtaattg tcattagatc acctgtgttg 1260
cagataggat cactctttat cattactgct gtaaaccctg ctacaattgg aagagaaaag 1320
 
gatatggaac atactctccg aatccctgaa gttggtgtca gtaagtttca tgcagaaatt 1380

tattttgacc atgacttaca aagttatgtc cttgtggatc aaggcagtca aaatggcaca 1440

attgttaatg gaaaacagat tcttcagccg aaaactaaat gtgaccctta cgtacttgag 1500

catggagatg aagtcaaaat tggagaaact gtcttatcct ttcacattca tcctggcagt 1560

gatacctgtg atggctgtga accagggcag gttagagccc accttcgcct tgataagaaa 1620

gatgaatctt ttgttggtcc aacactaagt aaggaggaaa aagagttgga aagaagaaaa 1680

gaattaaaga aaatacgagt aaaatatggt ttacagaata cagaatacga agatgaaaag 1740

acattgaaga atccaaaata taaagataga gctggaaaac gtagggagca ggttggaagt 1800

gaaggaactt tccaaagaga tgatgctcct gcatctgttc attctgaaat tactgatagc 1860

aacaaaggtc ggaagatgtt ggagaagatg ggttggaaga aaggagaggg cctggggaag 1920

gatggtggag gaatgaaaac gccgatccag cttcagcttc ggcgaacaca tgcaggcttg 1980

gggacaggca aaccatcctc atttgaagat gttcaccttc tccaaaacaa gaacaaaaaa 2040

aactgggaca aagcacgaga gcggtttact gaaaacttcc cagaaactaa gcctcaaaaa 2100

gatgacccag ggaccatgcc ttgggtaaaa gggactttag agtga 2145
   Sequence ID: 3
      Length: 32
      Molecular Type: DNA
      Characteristic Location/Qualifier:
         - source, 1..32
            > mol_type, other DNA
            > organism, synthetic construct
      Residues:
         gcggatccat ggcctcggag gcgccgtccc cg 32
   Sequence Number (ID): 4
      Length: 32
      Molecular Type: DNA
      Characteristic Location/Qualifier:
         - source, 1..32
            > mol_type, other DNA
            > organism, synthetic construct
      Residues:
         ccaagctttc actctaaagt cccttttacc ca 32
      END

## Claims

1. A use of a recombinant protein, **characterized in** being applied for preparing a drug for inhibiting an aneurysm; an amino acid sequence of the recombinant protein is as follows:
(4) as shown by an amino acid sequence of SEQ ID NO: 1; or
(5) an amino acid sequence having 80% to 100% homology with the amino acid sequence defined by SEQ ID NO: 1, and retaining amino acids at positions 606-608, wherein the amino acid sequence encodes a protein with a same function; or
(6) the amino acid sequence defined by SEQ ID NO: 1 that has undergone addition, deletion, or substitution of one or more amino acids, and that retains the amino acids at positions 606-608, possessing an equivalent activity to the protein shown in the sequence of SEQ ID NO: 1.

2. A use of a composition of a recombinant protein with a TGF-β inhibitor, **characterized in** being applied for preparing a drug for inhibiting an aneurysm; an amino acid sequence of the recombinant protein is as follows:
(4) as shown by an amino acid sequence of SEQ ID NO: 1; or
(5) an amino acid sequence having 80% to 100% homology with the amino acid sequence defined by SEQ ID NO: 1, and retaining amino acids at positions 606-608, wherein the amino acid sequence encodes a protein with a same function; or
(6) the amino acid sequence defined by SEQ ID NO: 1 that has undergone addition, deletion, or substitution of one or more amino acids, and that retains the amino acids at positions 606-608, possessing an equivalent activity to the protein shown in the sequence of SEQ ID NO: 1;
preferably, the TGF-β inhibitor is pirfenidone.

3. The use according to claim 1 or 2, **characterized in that** the aneurysm is an aortic aneurysm or a carotid artery aneurysm.

4. The use according to claim 1 or 2, **characterized in that** the aneurysm is an aneurysm caused by aortic arch coarctation.

5. The use according to claim 1 or 2, **characterized in that** the aneurysm is an aneurysm caused by FBN1 gene mutation.

6. The use according to claim 1 or 2, **characterized in that** the aneurysm is a thoracic aortic aneurysm.

7. The use according to claim 1 or 2, **characterized in that** the recombinant protein inhibits the aneurysm through inhibiting TGF-B1 synthesis and a signaling pathway thereof.

8. The use according to claim 7, **characterized in that** the signaling pathway is TGFβ1-TGFβR1-Smad2/3 and TGFβ1-TGF(3R1-ERK1/2.

9. The use according to claim 1 or 2, **characterized in that** the recombinant protein inhibits the aneurysm through improving a structure and functions of blood vessels.

10. The use according to claim 1 or 2, **characterized in that** the deletion of the amino acids at the positions 606-608 disrupts an interaction between an AGGF1 protein and a receptor integrin α7 thereof, thereby eliminating an effect of the AGGF1 protein.
